Europäisches Patentamt

European Patent Office

Office européen des brevets

(11) Publication number: 0 142 905
A2

# EUROPEAN PATENT APPLICATION

(21) Application number: 84302218.7

(22) Date of filing: 30.03.84

(51) Int. Cl.⁴: A 61 K 39/385
A 61 K 39/44, A 61 K 37/48

(30) Priority: 30.03.84 US 481280
26.09.83 US 536001
23.03.84 US 591330

(43) Date of publication of application:
29.05.85 Bulletin 85/22

(84) Designated Contracting States:
CH DE FR GB LI

(71) Applicant: BIO-RESPONSE INC.
550 Ridgefield Road
Wilton Connecticut 06897(US)

(72) Inventor: Rose, Sam
2701 Claremont Blvd. Berkeley
California 94705(US)

(74) Representative: Marsh, Roy David et al,
Brewer & Son 5-9, Quality Court Chancery Lane
London WC2A 1HT(GB)

(54) Therapeutic substance for treating diseases such as cancer.

(57) There is disclosed a substance for the *in vivo* treatment of a diseased cell population in a patient comprising an immunological complex which is adapted to serve as a precursor of a cytotoxic attack on a target cell of a cell population of heterogeneous diseased cells of a patient by being internalized by a disease cell by endocytosis a carrier substance which is immunospecific to a target diseased cell of the diseased cell population, and a non-metabolizable antigen complexed to the carrier substance, the non-metabolizable antigen being formed of antigenic material which is non-cytotoxic to a target cell, which is not broken down by any system or material to which it is exposed in a patient, and which is adapted to be de-coupled when the complex is internalized within the target cell, whereby the non-metabolizable antigen is adapted to be accumulated in a target cell by endocytosis induced by the carrier substance. The substance is useful in a method for treating a heterogeneous cell population in a patient, particularly a cancer cell population, wherein areas of exploitable mecrohomogeneity within the population are used as a springboard or base for developing an aggressive and extensive attack throughout the entirety of the tumor cell population.

Fig. 7.

THERAPEUTIC SUBSTANCE FOR TREATING DISEASES SUCH AS CANCER

This application is a continuation-in-part of my commonly-assigned, pending U.S. patent application Serial No. 536,001, filed September 26, 1983, entitled "Therapy Method", which in turn is a continuation-in-part of my commonly-assigned pending U.S. patent application Serial No. 481,280, filed March 30, 1983, also entitled "Therapy Method".

The invention of this application, as well as the inventions described in the above-noted earlier applications, relate to the general field of medical therapy and, in particular, to the field of medical immunotherapy. In its most immediate and important field of applicability, the invention relates to the treatment of cancer.

The starting point for the invention described herein and in my previous applications is the recognition of major deficiencies in current approaches to medical immunotherapy, particularly cancer immunotherapy.

The art is replete with discussions regarding the nature and mechanism of cancer cell formation, division and proliferation and with a variety of proposed therapies proceeding therefrom. As is apparent, the key to these problems and an understanding of the body's inability to deal with cancer through the normal immune defense system remain, to date, largely unknown.

What is known, of course, is that cancer cells can be killed and/or inactivated by, for example, certain drugs, poisons or irradiation or through endogenous complement- or cellular- mediated mechanisms. Many of these techniques have been demonstrated in *in vitro* studies and, in certain cases (e.g., radioiodide I-131 treatment of thyroid cancer) have been demonstrated *in vivo*.

In general, however, the *in vivo* treatment of cancer through use of cytotoxic agents has been extraordinarily unsuccessful. In the first place, of course, except in very rare circumstances, many of the agents which are cytotoxic to cancer cells are not "cancer-specific", i.e., they are equally, if not more, lethal to non-cancerous, normal cells. Administration of such agents to a patient exhibiting a cancer is fruitless in view of the host of undesirable side-effects brought about by their action on normal cells.

A far more promising approach to *in vivo* cancer therapy is based upon the "targeted" delivery of cytotoxic agents specifically, or generally specifically, to cancer cells. The art is replete with investigations attempting to identify some "marker" uniquely associated with malignant cells (or cells undergoing transformation to malignancy) which is not possessed by normal cells, be it a cancer-associated antigen, a particular surface receptor, a gene, or the like, in hopes of then utilizing this marker as a target for the cancer-specific delivery of a cytotoxic agent.

One of the more common approaches along these lines is the attempted development and isolation of cancer-specific antibodies, i.e., immunoglobulins which will exhibit specific binding affinity for some antigen or antigenic receptor uniquely associated with cancer cells. These antibodies might be cytotoxic per se to cancer cells or simply be used as specific vehicles for delivering a carried cytotoxic agent. To this end, malignant cells or organisms or antigens are introduced into a living host (e.g., rabbit, mouse) in order to stimulate the immune defense system of the animal into production of antibody against some antigen associated with the cancer. The difficulty with this approach has been that, even if a uniquely cancer-specific antibody can be generated, it will be produced in association with a considerable number of other antibody products generated by the animal in response to the inoculation. The isolation of the particular desired antibody from the mixture of cellular products obtained involves an extraordinarily tedious and lengthy process of separation, purification, testing, and the like and, even then, may not result in isolation of an antibody of sufficient purity to guarantee specific interation only with cancer cells.

With the advent of cell hybridization techniques in the mid-1970's as a means of producing highly specific "monoclonal" antibodies, considerable enthusiasm was generated among immunoncologists that perhaps the long-sought "magic bullets" against cancer cells had been found. In these techniques, antibody-producing cells, obtained from an animal or human subject exhibiting a cancer or deliberately inoculated (in vivo or in vitro) with cancer cells or antigens, are hybridized to cells  capable of continuous division (e.g., myeloma cells). Hybrid cells resulting from the in vitro fusion of a single myeloma cell and a single antibody-producing cell can produce only a single antibody product and, owing to the immortality of the myeloma parent cell, can produce this antibody product for long periods of time. Hence, product isolated from a colony of cells grown up from a hybrid cell is uncontaminated by other cellular products and, by reason of such purity, has the capability of exhibiting a high degree of immuno-specificity for a particular antigen. By testing the product from each of the monoclonal hybridoma colonies produced in this manner, it is possible to find one or more monoclonal antibodies which exhibit specific or substantially specific affinity for antigens or antigenic receptors associated with cancer cells, which antibodies can be used per se or as vehicles for directing cytotoxic agents to cancer cells.

Notwithstanding these advances in antibody production, the optimism they originally engendered has not borne out in practice and the immunotherapeutic treatment of cancer based upon cancer-specific monoclonal antibodies has been far less successful than expected. To a certain degree, this lack of success can be attributed to problems associated with the making and testing of monoclonal antibodies. More importantly, however, the basic scheme of therapy itself using cancer-specific monoclonal antibodies is fundamentally flawed.

In my previous U.S. patent applications, Serial Nos. 481,280 and 536,001, the reasons for the lack of success which characterizes current approaches to immunotherapy are elucidated, and unique methods for dealing with those shortcomings are provided. The present application provides still further explanation and expansion of unique approaches to immunotherapy, particularly as applied to cancer immunotherapy.

The most serious deficiency of current cancer immunotherapy techniques is that they do not take into account the heterogeneity[1] which exists within a tumor cell population, i.e., the very different nature, characteristics, composition, functions and properties of the many individual cancer cells making up the tumor cell population. Taking antigenic cell receptors as an example, it will be found that the individual cancer cells of the tumor population do not all exhibit the same array of receptors. Thus, even were it possible to find or develop a cytotoxic antibody or ligand (or antibody or ligand capable of carrying a cytotoxic agent) which exhibits immunological specificity for a cell receptor unique to cancer cells (i.e., not possessed by non-cancerous cells), the ligand will not attach to every cancer cell in a tumor population because not every cancer cell exhibits the particular receptor. Accordingly, while the immunotherapeutic administration of cytotoxic agent by means of immunospecific ligands (e.g., antibodies) might successfully localize treatment to cancer (vs. non-cancer) cells, the therapy is nevertheless ineffective because not all the cancer cells will be treated and will simply continue to proliferate. Indeed, as more and more specificity is sought as between cancer-versus non-cancer-specific agents, the likelihood that the agent will affect all possible cancer cells in a tumor population becomes less and less. In theory, of course, it might be possible to develop an entire array of cytotoxic immunospecific ligands to insure that at least one such ligand will attach to every cancer cell in the

---

[1]    Sprenulli and Dexter, Journal of Clinical Oncology, Vol. 1, No. 8, August, 1983.

population; however, such an approach, even if possible, would involve the expenditure of an enormous amount of time and effort to perform all the experimentation and testing required to develop such an array. Moreover, as the number of ligands to be administered to gain such insurance increases, so too does the possibility that receptors for some of these cytotoxic ligands will be found to exist on non-cancerous cells. Still further, and perhaps most importantly, such an approach necessarily involves greatly increasing the burden on the patient in terms of the absolute quantity of cytotoxic agent which is circulating throughout the patients bloodstream.

This cancer cell population heterogeneity exists with respect not only to antigenic cell receptors but also with respect to every other measurable parameter conceivable, e.g., drug sensitivity, gene amplification, differentiated function, growth rate, chromosome number, size, buoyancy, etc. Even were it possible to distinguish normal from cancerous cells based upon any of these parameters, the cancerous cells per se can be shown to be heterogeneous as to the chosen parameter.

Another very serious shortcoming of those immunotherapy techniques which rely upon the specific delivery of cytotoxic agents by means of immunospecific ligands (e.g., antibodies) is that there are very definite limits on the quantity of cytotoxic agent which can be brought to the cancer cell in this way. Thus, for example, the absolute quantity of cytotoxic agent which can be affixed to an immunospecific antibody, without altering the specificity of the antibody, generally is significantly less than the amount of agent required to kill a cancer cell. Some damage to the cancer cell may occur, but, owing to the plasticity of cancer cells, the damage often is only temporary or may simply allow the emergence of mutant cells which are still cancerous but are resistant to the effects of the cytotoxic agent.

A further difficulty which is inadequately dealt within current immunotherapeutic approaches to cancer is the fact that the rate at which macromolecules (e.g., antibodies or other ligands, with or without carried agents) are transferred from the blood to cells is extremely slow, and is particularly slow with respect to cancer cell populations. This low permeability of capillaries to such macromolecules in general, coupled with the low levels of vascularization in solid tumors, makes delivery of cytotoxic agents to tumor cells problematical. Additional difficulties involve the dynamics of circulating antibodies in the body (making it difficult to cause an antibody-carried cytotoxic agent to remain in the vicinity of tumor cells for a sufficiently long time to effect aggressive killing) and the slow rate of decay of particular cytotoxic agents (e.g., radioactive material) from the blood.

It will be seen, therefore, that the current approaches to cancer immunotherapy involve a number of counter-productive and counter-balancing features which severely limit their utility. On the one hand, the primary goal is to predicate the therapy upon a basis whereby a cytotoxic attack is, insofar as possible, confined only to cancer cells and will not affect normal cells. Otherwise, the proposed therapy is doomed to begin with. At the same time, however, the very specificity which is sought to be attained for achieving the requisite cancer cell versus non-cancer treatment literally insures that the treatment as to cancer cells will be incomplete and ineffective, since not all the cancer cells of the population will exhibit specificity for the chosen treatment. Moreover, even as to those cancer cells in the population which are suitable for the chosen treatment (e.g., which will in fact bind a particular ligand or ligand-cytotoxic agent complex), the treatment may not result in the killing even of these cancer cells, let alone other cancer cells in the population, In addition, the task of getting macromolecules to travel through the circulatory system to some desired site is considerable.

In accordance with the present invention, there is provided a methodology for the treatment of cancer cell populations, as well as for the treatment of other diseased cell populations, which overcomes the many shortcomings of techniques currently in use.

As will be apparent from the description which follows, the method of the present invention essentially combines inherently immunospecific and non-immunospecific steps in a manner whereby treatment is localized within a particular population but, as to the population per se, the treatment is non-specific and pervasive. In this way, a cytotoxic attack can be preferentially localized, within a patient having non-cancerous and cancerous cells, to the areas where the cancer cells exist, thereby minimizing (and, ideally, eliminating) any adverse effects on populations of normal cells. Once localized to the particular area, however, the cytotoxc attack is then arranged to be non-specific and aggressive within the area, thereby insuring that all cancer cells in the area are killed.

In order to more fully understand the approach employed in the present invention, it is useful to refer to the following illustrative embodiment. Consider, then, a patient having a cancer tumor in a particular area of the body. In the known immunotherapeutic cancer treatment, as well as in the present approach, the goal is to localize treatment to the area of the tumor mass, leaving undisturbed all other areas of the patient. In the known method, this is accomplished by development or isolation of, e.g., an antibody having specific affinity for a particular receptor unique to a cancer cell (i.e., in the sense of not being possessed by a non-cancerous cell). This antibody, which is _per se_ cytotoxic or which carries a cytotoxic agent, is therefore preferentially directed to the tumor mass wherein such cancer cells exist, and will not be directed to any other areas in the body.

As earlier mentioned, however, the major problem with this known method is that the killing of cancer cells is directly dependent upon the binding of the antibody to the particular receptor. Because of this, it may not be possible to bring a sufficient killing quantity of cytotoxic agent to those cancer cells where such binding occurs. More importantly, however, the heterogeneity of the tumor mass means that only a portion of the cancer cells within the tumor mass will exhibit the specific receptor binding site for the antibody and thus, even if killing is effected, it is only this small portion of cancer cells which will be killed. All other cancer cells within the tumor mass will continue to proliferate.

In the present invention, immunospecific agents (e.g., antibodies) are also employed as a means of localizing treatment to the area of the tumor cell population; however, in marked contrast to known techniques, this cancer-specific step is not relied upon to effect the complete killing of cancer cells. Rather, the cancer-specific step involves non-cytotoxic materials and is used merely as a springboard or stepping-stone for thereafter causing to occur an aggressive and extensive cytotoxic attack which, as to the tumor cell population, is non-specific. In this way, the heterogeneity of the tumor cell population is no longer problematic. So long as at least some cancer cells in the tumor cell population can be targeted for specific binding of a ligand (antibody), that binding can then be used as a base for achieving aggressive, widespread killing within the tumor mass.

The foregoing constitutes the key feature of the present invention, i.e., the treatment of a diseased (e.g., cancerous) cell population, heterogeneous from cell to cell with respect to therapeutically exploitable parameters but containing at least some (target) cells as to which at least one such parameter is substantially unique vis-a-vis non-diseased cells, by directing an agent specifically to such target cells and using the agent to set in motion a cascading sequence of steps designed to cause, within the diseased cell population, an extensive, aggressive, non-specific cytotoxic attack.

In the process of the present invention, the specific binding of a ligand to a receptor on a target cell is used as a base upon which to develop an amplified therapeutic (cytotoxic) attack throughtout the area of the affected cell population. By means of the amplification, many more molecules of therapeutic or cytotoxic agent can be brought to bear upon the cell population than is possible where the delivery of such agents is directly tied to ligand/receptor binding.

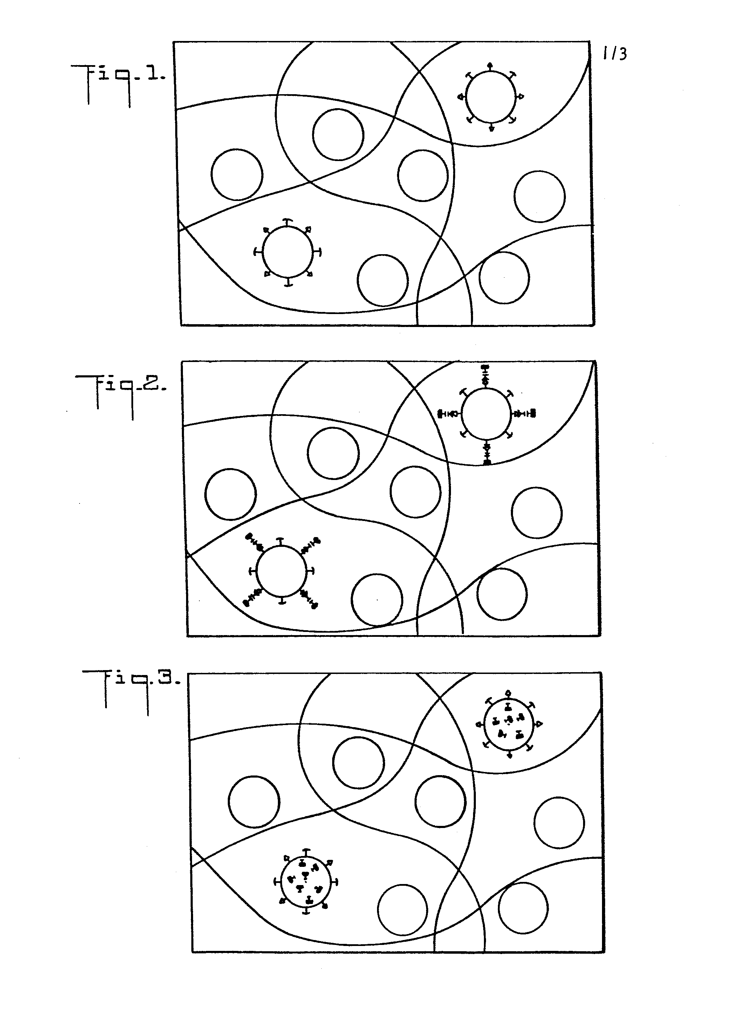

FIGS. 1 through 7 are schematic representations of a tumor cell population in the body and the sequence of steps involved in the execution of the preferred embodiment of the therapy method of the present invention.

In the following description of the invention, emphasis is placed upon the applicability of the invention to the treatment of cancer cell populations, since in this environment the distinctions between the approach taken herein and that of the prior art, and the considerable advantages obtained thereby, are most readily and easily perceived; however, the process of the present invention is not limited to cancer therapy and has wide applicability to a variety of therapeutic treatments of diseased cell populations wherein treatment is sought to be localized to the diseased population, but wherein the heterogeneity of the diseased population otherwise hinders the proposed therapy.

In accordance with the present invention, a number of methods are presented for dealing with the shortcomings of known techniques for the immunotherapeutic treatment of cancers in living subjects. At the heart of each method is the exploitation of some characteristic(s) of cancer cells which distinguishes them from non-cancerous cells, while recognizing that such characteristics may not be exhibited by every cancer cell in a tumor population. The "exploitation" involves using such characteristic as a means of specifically directing a precursor of a cytotoxic attack to a tumor cell population, as opposed to other cell populations in the body where tumor cells do not exist.

For purposes of describing the present invention, there is first discussed what is considered at present to be a preferred method for executing the invention. This method is deemed "complex" herein in the sense of attempting to focus on, and solve, all potential difficulties in the treatment of cancer with cytotoxic agents. As will be apparent, however, the degree to which all such difficulties are dealt with need not always be as extensive as described in this preferred method. Accordingly, many other procedures of somewhat less complexity also are described hereinafter.

In the preferred method, an immunospecific ligand is employed as a carrier for a non-metabolizable "antigen", i.e., an antigenic material which will not be digested and broken down by any system or material to which it will be exposed in the patient, be it a body fluid or the surface or internal mechanism of cancerous and non-cancerous cells. In marked contrast to the methods proposed in the art, this ligand/non-metabolizable antigen complex is not cytotoxic.

The preferred ligand is a monoclonal antibody isolated from a hybridoma mass which has been grown up from hybrid cells produced by hybridizing a cell line capable of continuous division to antibody-producing cells harvested from a mammal which has been innoculated with a particular antigen or antigen-bearing cells or organism or which is diseased in a manner which exhibits the antigen in question.[2] As earlier noted, such monoclonal antibodies can be made which are cancer-specific in the sense of exhibiting substantially specific affinity only for a cancer cell. Due to the heterogeneity of cancer cell populations, however, the monoclonal antibody will not exhibit affinity for every cancer cell in the population. For purposes of the present invention, this heterogeneity is not disadvantageous. All that is required is that the tumor cell population exhibit one or more areas of exploitable

2

microhomogeneity, i.e., in this case one or more cancer cells ("target cells") which do specifically bind the monoclonal antibody.

The complex[3] of monoclonal antibody and non-metabolizable antigen[4] is such that it will be internalized by those target cancer cells exhibiting affinity for the antibody, through the mechanism of ligand-induced endocytosis.

In most instances, the discharge of a ligand from its receptor can be triggered by mildly acidic pH. That this mechanism operates intracellularly has been clearly demonstrated. Within minutes after internalization in pinocytic vesicles, receptor-ligand complexes are delivered to a second class of vesicle referred to as endosomes. It has been shown that the endosome membrane contains a proton pump which can lower the internal endosome pH to 5.2. Thus, the endosome is the site of receptor-ligand dissociation and the site from which free receptors recycle to the cell surface. In contrast, discharged ligands remain inside the endosome which then transports them to hydrolase-rich lysosomes, where they either are accumulated or (if sensitive to proteolysis) are degraded. Since most antibodies do not dissociate from their antigens at endosome pH, they are not normally accumulated by cells in this way; however, several modifications are possible which would allow antibodies (or molecules bound to them) to behave more like physiological ligands in this respect.

The movement of dissociated ligands to

---

[3] Conjugates Bumol et al, Proceedings of National Academy of Science, Vol. 80, p. 529, 1983.

[4] Polysaccharides, e.g. Dextran or D-Amino Acid Peptide) lysosomes is largely unidirectional. Unlike the situation in endosomes, there is litle recycling of membrane from lysosomes to the cell surface. Thus, transport from endosomes to lysosomes is a sine qua non for the intracellulr accumulation of any cytotoxic system. Monoclonal antibodies which do not dissociate from their antigens in endosomes will not normally reach lysosomes and, consequently, will not be accumulated by most tumor cells, however, it has been found that it is possible to induce the transport of intact antibodyantigen complexes to lysosomes by slightly altering the chemical nature of the antibody employed.

Apparently, the change required involves increasing the antibody's valency, i.e., its number of antigen binding sites. Using a macrophage-like tumor cell line, test data indicate that while monovalent antibody is internalized, delivered to endosomes, and recycled, polyvalent complexes are rapidly and irreversibly transferred to lysosomes. This alteration in membrane traffic occurs because polyvalent complexes can cluster or aggregate individual antigen molecules in the cell membrane. Membrane antigens clustered in this way are prevented from recycling, and thus are effectively trapped intracellularly.

Low pH-mediated discharge and polyvalent complex-induced clustering represent two simple yet effective strategies to cause the intracellular accumulation of antibody-conjugated cytotoxic systems. It is already clear that either of these approaches can in principle be used to deliver the required amount of antibody to tumor cell lysosomes. Since low pH-mediated delivery involves the repeated use of individual plasma membrane antigens, the maximum amount of antibody which can be delivered is virtually without limit. Many specific cell surface receptors are known which use this strategy to effect the continuous internalization accumulation of hundreds of thousands of ligand molecules every hour. On the other hand, the amount of antibody conjugate which can be accumulated by induced clustering is partly controlled by the number of copies expressed of a particular antigen. In general, this approach involves the one-time use of a cell surface antigen which is internalized and delivered to lysosomes along with its bound antibody. Nevertheless, a test system using a reticulum sarcoma shows that $> 10^6$ antibody molecules can be induced to accumulate lysosomes within 1 hour.[5]

The antibody, and the means for complexing non-metabolizable antigen to the antibody, are chosen so that once internalized into the target cell, either (a) the complex dissociates (e.g., by virtue of a pH effect utilizing the low pH in the endosome region of the cell or by an enzymatic-effect using the enzymes in the lysosome region of the cell) and the antibody de-couples from the receptor and/or (b) the antibody de-couples from the receptor with the non-metabolizable antigen intact. In either event, the intent is to accumulate many molecules of non-metabolizable antigen within the target cell over a period of time (accumulation of as many as 0.5 X $10^6$ molecules per hours of particular ligands by ligand-induced endocytosis in tumor cells is possible). By definition, the non-metabolizable antigen will not be broken down by the target cell once internalized.

In order to achieve the desired accumulation,

[5] (a) Pearce and Bretscher, Annual Review of Biochemistry, Vol. 50, P. 85 (1951); (b) Pastan and Willingham, Annual Review of Physiology, Vol. 43, P. 239 (1981); (c) Galloway et al, Proceedings of the National Academy of Sciences, Vol. 80, P. 3334 (1983); (d) Helenius et al, Trends in Biochemical Science, Vol. 8, P. 248 (1983) the interaction between the monoclonal antibody and the specific receptor on the target tumor cell surface should be non-modulating, i.e., after antibody or antibody/antigen complex is de-coupled from the receptor, the cell should rapidly re-cycle the receptor back to the cell surface to act as a mediator for the next internalization of antibody and non-metabolizable antigen so that continued accumulation can occur; however, a distinct advantage of the process of the present invention is the ease with which even modulating systems can be dealt with. In such systems, the particular surface receptor eventually will reappear on the cell surface, and all that is required is that the infusion of antibody-coupled non-metabolizable antigen be held up until such reappearance occurs. That this can be done is a direct consequence of an important feature of this embodiment of the invention, i.e., that the material sought to be accumulated in the target cell (non-metabolizable antigen) is not cytotoxic.

In addition, the half-life of non-metabolizable antigen with the target cell is very long, generally greater than 101 days. Hence, non-metabolizable antigen can be accumulated in a target cell via ligand-induced endocytosis for however long the surface receptor which mediates the endocytosis is present. If the receptor disappears, one simply waits until it reappears before administering additional antibody-coupled non-metabolizable antigen, the already accumulateed antigen simply staying stably in place in the target cell.

Since the non-metabolizable antigen is not cytotoxic, there is no burden on the patient as there would be if a cytotoxic poison were permitted to circulate in the bloodstream while awaiting complete endocytosis of a desired quantity thereof.

As will be apparent in discussion of further embodiments later in this application, another means to deal with modulating systems is simply to use a number of different antibody carriers for the non-metabolizable antigen. As one particular receptor for a particular antibody-coupled non-metabolizable antigen disappears, a second different antibody is then used to carry in the same non-metabolizable antigen, this time mediated by a different surface receptor. This sequential process can be carried on using different antibodies until such time as an earlier-used receptor reappears on the cell surface. Again, the long half-life of the non-metabolizable antigen accumulated in the target cell, and the non-cytotoxicity of the non-metabolizable antigen, makes this possible.

Continued administration of the monoclonal antibody non-metabolizable antigen complex thus results in continued accumulation of non-metabolizable antigen within particular target tumor cells of the tumor cell population. As has been noted, since the agents employed in this step are not cytotoxic and are not being relied upon to effect killing, the administration regimen can be conducted over however long a period is required to get these molecules through the capillary system to the tumor cells and to induce internalization of a large number of molecules of non-metabilizable antigen in target tumor cells.

After accumulation of a large number of molecules of non-metabolizable antigen, "free" monoclonal antibody, non-metabolizable antigen or complexes thereof which have not been internalized by target tumor cells are eliminated from the blood and lymph streams through procedures such as plasmaphoresis, lymphoresis, and the like. Again, although these procedures can be time-consuming, time is not of any

-18-

particular concern at this stage since the accumulated non-metabolizable antigen, which is the predicate for the subsequent steps in the process, will remain in the target cancer cells until specific steps are taken to release it.

Elimination of "free" non-metabolizable antigen (or complexes thereof with monoclonal antibody) form the kidney and from the reticulo-endothelial system (RES) is potentially a more difficult and problematical task. One means for dealing with at least the problem of undesired accumulation in the kidney is to infuse a microbial[6] or plant enzyme which is capable of digesting the otherwise non-metabolizable antigen. Another means for dealing with both kidney and RES accumulation is to select as the non-metabolizable antigen a material which, when internalized by a cell, is de-coupled from its antibody carrier or otherwise altered (e.g., by lysosomes) in a way so as to expose an antigenic determinant which will not otherwise exist on non-metabolizable antigen which has not been internalized in cells. Since, as later explained, the binding of an antigenic determinant of the non-metabolizable antigen to a particular antibody therefor is an important subsequent step in the therapy method, such binding will not occur in areas such as the kidney or RES where internalization of the non-metabolizable antigen, and exposure of the determinant, has not occurred.

At this juncture of the process, it will be noted that an immunospecific step has been employed in order to obtain an accumulation of material (non-metabolizable antigen) in at least some of the cells in a tumor cell population. The step is immunospecific in the sense of preferentially effecting such accumulation only in tumor cells as opposed to non-cancerous cells,

---

[6]    Dextranase $\alpha$-1,6 Glucan 6-glucanohydrolase.

but it is not required (and, indeed would be impossible to achieve) that every cancer cell in the population accumulate the material.

This immunospecific step (after elimination of free material) is then followed by what is essentially a non-cancer-specific step involving the provision of a mechanism for capturing non-metabolizable antigen when, as later described, it is released from at least some of the target cancer cells in which it has accumulated. This capturing mechanism can take a variety of forms, but in the preferred method consists of a substantially systemic matrix material which is arranged to be present, insofar as possible, throughout the body, and which exhibits specificity for the non-metabolizable antigen.

A particularly preferred systemic matrix material consists of either a foreign fibronectin, a haptenated fibronectin or a fibronectin on which is carried antibody to the non-metabolizable antigen. Continuous infusion of such fibronectin over time will result in its covalent incorporation into the complex extracellular matrix of the body. Again, since this material is not cytotoxic, and since the non-metabolizable antigen previously accumulated in target tumor cells will remain there for long periods of time until released, the possibly lengthy period of time required to effect the systemic infusion of fibronectin throughout the body is not a problem.

Four plasma proteins are known to be present in an insoluble form in extracellular matrices; fibronectin (Oh et al, 1981), amyloid P component (Dyck et al, 1980), von Willebrand factor (Rand et al, 1980), and vitronectin (Hayman et al, 1983). The concentration of these four proteins in plasma are 300 ug/ml, 30 ug/ml, 10 ug/ml, and approximately 200 ug/ml, respectively. In addition, a protein called thrombospondin is released into serum from platelets during blood coagulation and incorporated into the extracellular matrix, at least in cell culture (McKeown-Longo, et. al., 1984).

Fibronectin can be easily purified in gram quantities from plasma or, for the human protein, from side fractions of Factor VIII purification (Mosher and Johnson, 1983). Haptens can be introduced into specific sites of homologous fibronectin, e.g., dansylcadaverine in a transglutaminase site (Williams et al, 1982) and IEDANS into free sulfhydryls (Mosher and Johnson, 1983), or into non-specific sites, e.g., derivatization of lysyl residues with trinitrophenol. The hapten-protein conjugate will be characterized in regard to its hydrodynamic properties (Williams et al, 1982), protease susceptibility (Mosher and Johnson, 1983), and ability to be incorporated into extracellular matrices of cultured cells (McKeown-Longo and Mosher, 1983).

In the next step of the process, the accumulated non-metabolizable antigen is released from at least some of the target tumor cells. This release can be effected by the lysis of these cells by means of endogenous complement[7] or antibody dependent killing.[8]

---

[7] (a) Complement Lysis - Mayer, Pnas Volume 69, p. 2954, 1972

(b) Mayer et al. - Critical Reviews in Immunology, Volume #, p. 133, 1981.

[8] (a) Antibody Dependent Cellular Toxicity Moller - Science, Volume 197, p. 873, 1965.

(b) Burnstein et al. - Science - Volume 207, p. 68, 1980.

(c) Hybridoma - Kohler and Melstein Nature, Volume
(footnote continued)

Thus, this step in the process is immunospecific for cancer cells in that it is not desired to kill any non-cancerous cells. Again, however, the requirement here is simply to lyse at least some cancer cells, some of which will be the target cells which have accumulated non-metabolizable antigen. It is not necessary to kill all the cancer cells in this step and, hence, the heterogeneity and plasticity of the tumor cell population need not be dealt with.

It is preferred that the antibody used in this step be a monoclonal antibody, exhibiting specificity for at least some characteristic of at least some of the tumor cells of the cell population, which is capable of mediating lysis without need for a cytotoxic agent being conjugated thereto.[9] The concentration of this monoclonal antibody will have to be high in order that enough of the antibody will seep or diffuse through the capillaries to establish the required concentration. The preferable monoclonal antibody is that secreted by the antibody producing cells of the patient to be treated which have been hybridized. The antibody producing cells can be obtained from the patient's tumor, blood, spleen, lymph nodes, etc. The antibody producing cells are preferably stimulated in vitro by incubating the antibody producing cells with the patient's own tumor cells or an extract of the tumor

---

(footnote continued from previous page)
256, p. 195, 1975.

(d) Kohler and Melstein - European Journal of Immology, Volume 6, p. 611, 1976.

(e) Margulies et al. - Cell, Volume 8, p. 405, 1976.

[9] Conjugates - Olsens et al. - Pharmacology Therapeutics, Volume 15, p. 355, 1982.

Thorpe and Ross - Immunological Reviews, Volume 62, p. 119, 1982.

cells containing the right antigen. See "Successful <u>In Vitro</u> Primary Immunization of Human Peripheral Blood Mononuclear Cells And Its Role In The Development of Human-Derived Monoclonal Antibodies", Joy Cavagnaro, Ph.D and Michael E. Osband, M.D., Bio-Techniques, P. 30-36 (1983).

By proceeding in this manner, the possibility of this step having an effect on non-cancerous cells is greatly diminished, particular as to cells of the RES which, even if they did take up the antibody-coupled non-metabolizable antigen, are generally not killed through the action of antibody and complement and, therefore, will not release non-metabilizable antigen.

In addition, it is preferred that the antibody used in this step be different from the monoclonal antibody used to initiate the cancer-specific ligand-induced endocytosis of non-metabolizable antigen, for in this way, greater specificity as between cancer and non-cancer cells can be achieved. Thus, for example, if the administraton of the first monoclonal antibody (coupled to non-metabolizable antigen) somehow results in the accumulation of non-metabolizable antigen in a non-cancerous cell (which for some reason exhibits a receptor for the antibody), then use of the same or similar antibody in the lysis step might result in lysis of this cell and release of non-metabolizable antigen in an area of the body where no tumor cells exist.

In another embodiment, the monoclonal antibody brings in an agent by endocytosis, the agent becomes decoupled by the lysosome enzymes which also enzymatically act on the agent[10] to convert the agent

---

[10]   Siu Cheong H., Leaf Huang, Journal of Histrochemistry and Cytochemistry, Vol. 31, P. 104 (1983).

into an insoluble material or precipitate. By way of
example, the agent can comprise a benzedene compound.
In some cases, the pricipitate will have a neo-antigen.
As a result the radio-labelled antibody will go to the
precipitate having the old antigen or the precipitate
having the new antigen. Whether the precipitate has the
old antigen or the neo-antigen on it, the precipitate
captures the radio-labelled antibody; however if the
precipitate has a neo-antigen on it as a result of its
intracellular sojurn while being acted upon by the
lysosome enzymes and if the radio-labelled antibody is
directed to the neo-antigen, then any inadvertent
deposition of the antibody conjugate into extracellular
locations, such as the kidney, will not cause a problem
since the radio-labelled antibody directed to the neo-
antigen will not attach since this location has no neo-
antigen.

Since the aggressive cytotoxic attack depends
upon the presence of released non-metabolizable antigen
(as later described), this situation would result in a
cytotoxic attack being brought to bear on a normal
population of cells in the body, an obviously undersired
result. By using two different cancer-specific
antibodies for the accumulation and lysis steps, the
possibility of the foregoing situation occurring is
greatly minimized since it is most unlikely that a
normal cell will exhibit receptors for both anitbodies.
By the same token, the mere fact that the antibody used
in the lysis step might kill a normal cell is not
problematic since the killed cell will not be one which
has accumulated non-metabolizable antigen.

When target cells which have accumulated non-
metabolizable antigen lyse, the non-metabolizable
antigen is released and binds to the ubiquitous
fibronectin capturing matrix previously introduced. In this way, a large number of molecules of non-metabolizable antigen become stably affixed in the vicinity of tumor cells and, hence, within the tumor cell population. Owing to the stability of the affinity complex between the matrix and the released non-metabolizable antigen, there is adequate time to eliminate from the body any unaffixed non-metabolizable antigen.

In the final step of the process, a ligand exhibiting specific binding affinity for an unoccupied determinant site on the non-metabolizable antigen (i.e., some determinant site other than that by which the non-metabolizable antigen is actually affixed to the fibronectin matrix, e.g., a different determinant on a multi-valent non-metabolizable antigen or the same determinant simply at a different site on the non-metabolizable antigen or a determinant exposed by the de-coupling of the non-metabolizable antigen from the monoclonal antibody in the interior of the target cell in which it was endocytosed) is coupled to a cytotoxic agent, such as molecules of I-131 or any other isotope. This ligand can specifically be designed to be small so as to cause it to rapidly pass through the capillaries to the non-metabolizable antigen fixed on the fibronectin matrix in the midst of a tumor cell population. In this way, a very large number of I-131 or other isotopes molecules become stably affixed in a tumor cell population where they then produce a highly aggressive, prolonged cytotoxic attack capable of extending beyond a particular cell in the tumor population so as to kill all cells in the tumor population.

The foregoing process is schematically illustrated in FIGS. 1 through 7. FIG. 1 shows a population of cancer cells (represented by circles) existing within the normal connective matrix of the body. At least some of these cancer cells exhibit a surface receptor (represented by —→ ) which is unique to cancer cells, i.e., which is not generally possessed by a non-cancerous cell. In addition, some of the cells also possess a different receptor (represented by —→ ) unique to cancer cells. Some of the cells exhibit both receptors.

It is possible to produce and isolate monoclonal antibodies which will exhibit specificity for these particular receptors, which antibodies may be cytotoxic per se or can carry a cytotoxic agent. However, even if these antibodies or complexes can result in killing of a cell to which they affix, the problem is that not all cancer cells in the population exhibit the required receptor or receptors, and the killing of particular cells will have no effect on these neighboring cancer cells.

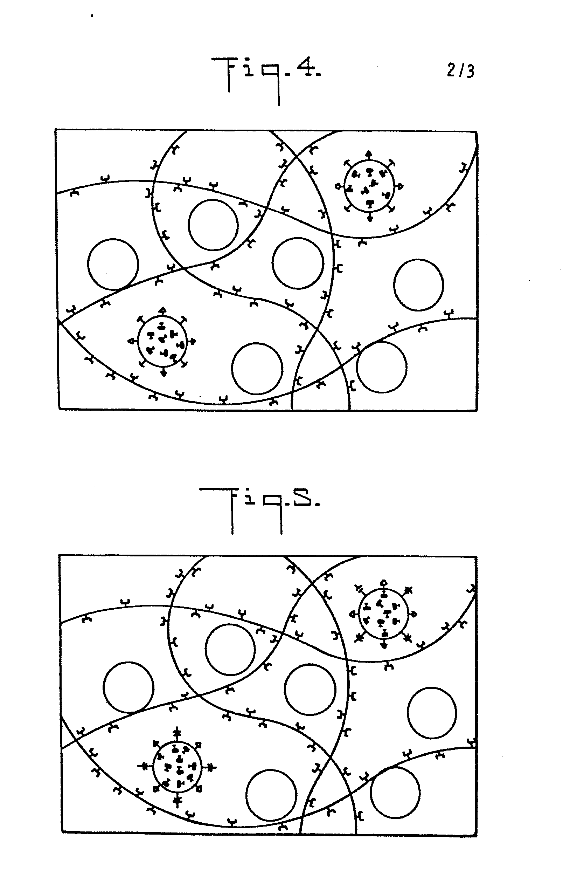

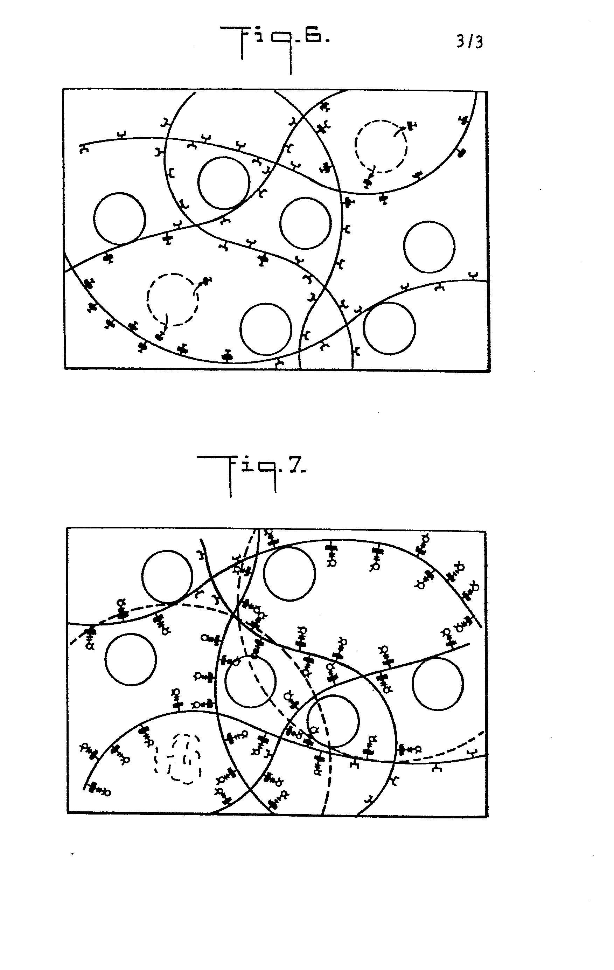

In accordance with the preferred embodiment of the invention, therefore, the first step of the process (see FIG. 2) is to administer to the patient monoclonal antibody ( —< ), specific to an expressed receptor, on which is coupled a non-metabolizable antigen ( ⊢▪ ) which is either multi-determinant or on which an antigenic determinant is exposed when de-coupling from the monoclonal antibody occurs. The monoclonal antibody/non-metabolizable antigen complex affixes to the receptor on at least some of the tumor cells of the tumor cell population and is then internalized by these (target) cells by ligand-induced endocytosis (see FIG. 3), whereby the non-metabolizable antigen is decoupled from the antibody and continues to accumulate within the target cells.

In the next step of the process, after elimination from the patient of free, non-accumulated non-metabolizable antigen, the matrix material, bearing a mechanism (ᴈ— ), for capturing non-metabolizable antigen, is infused into the patient (see Fig. 4). The matrix, e.g., foreign or haptenated or derivatized fibronectin, is systemic and incorporates itself throughout the normal fibronectin matrix of the body.

Thereafter (see FIGS. 5 and 6), an unconjugated monoclonal antibody (—ᴄ ) to a receptor different from that exploited in the first step is administered. This antibody mediates the lysis of at least some cells in which non-metabolizable antigen previously was accumulated, relaesing the non-metabolizable antigen for capture by the matrix material. It will be seen that at this stage of the process (after elimination of unbound non-metabolizable antigen), the therapy method no longer involves the cells of the tumor population; rather, the preceeding steps now result in the localization within the tumor cell population of stable, affixed non-metabolizable antigen. It is this antigen, now present in large quantity only in tumor cell populations, which then forms the base for the cytotoxic attack. By proceeding in this manner, many more molecules of cytotoxic agent are brought to bear on the tumor, the heterogeneity of the tumor cells is no longer a limitation, and the need for macromolecular cytotoxic antibodies or antibody/cytotoxic agent complexes is eliminated. Thus, in the final step (FIG. 7), many molecules of an I-131 labelled, small ligand ( ⊢ɑ ) which exhibits specificity for an exposed determinant on the captured non-metabolizable antigen are administered. These molecules rapidly become affixed in the matrix where they can remain for extended periods. As a consequence of the large number of molecules and the duration of stable affixation, the cytotoxic attack brought about by

0142905

-27-

these radioactive molecules is highly aggressive and widespread, resulting in the outright killing of all cells in the tumor population.

The foregoing detailed process demonstrates the fundamental concept of the present invention in overcoming the limitations and errors in known approaches to cancer immunotherapy. As earlier noted, many variations are possible within this fundamental concept.

In accordance with one of these alternative processes, non-metabolizable antigen is similarly accumulated in one or more target cells by ligand-induced endocytosis, mediated by a cancer-specific monoclonal antibody. After a sufficient quantity of non-metabolizable antigen has been so accumulated, free monoclonal antibody, non-metabolizable antigen or complexes thereof can be eliminated through plasmaphoresis, lymphoresis and/or use of immunosorbents or complexing agents.

In the next step in this particular process, an antibody (or other ligand exhibitng binding affinity) to an antigenic determinant or binding site on the non-metabolizable antigen is introduced into the patient. For this particular embodiment, the antibody has coupled to it an enzyme, the function of which is explained hereinafter.

The next step in the process is to release accumulated non-metabolizable antigen from at least some of the target cells in the manners described earlier with respect to the preferred embodiment.

Upon lysis of target cells, the many molecules of released non-metabolizable antigen will complex with the previously introduced antibody therefor (carrying an enzyme) and, having suitably chosen both antigen and antibody, form a precipitate in the vicinity of the lysed cells. In this immobilized precipitate, the enzyme carried by the antibody for the non-metabilizable antigen is bound and can then be used, either directly or indireclty, to bring about an aggressive, extensive cytotoxic attack throughout the tumor cell population. The attack is extensive both because it is nonspecific within the tumor cell population and because many more molecules of cytotoxic agent can be generated in the population than could be brought to bear on the cells by means of simple targeting of a cytotoxic agent plus immunospecific carrier. For example, if a substrate is used which is converted to a cytotoxic material by the enzyme at a turnover rate of $10^3$ molecules per second, the $10^6$ molecules of enzyme stably maintained in the precipitate (y lysis of one target cell containing $10^6$ molecules of non-metabolizable antigen which bind to $10^6$ molecules of enzyme-bearing antibody) will generate $10^9$ molecules of cytotoxic agent every second.

Within the context of the foregoing embodiment, many other variations are possible. Thus, for example, the antibody for the non-metabolizable antigen need not carry enzyme. Rather, an enzyme-carrying antibody to the precipitate formed from the non-metabolizable antigen and its antibody can be used to stably affix enzyme in the tumor cell population. In a preferred technique, the enzyme carried by the antibody for the non-metabolizable antigen is used to convert a substrate to a neo-antigen. Antibody specific to the neo-antigen can then be employed to carry many molecules of cytotoxic agent to the tumor cell population or to simply enlarge the precipitate, after which an antibody to the precipitate, carrying cytotoxic agent, or a precursor thereof, is introduced.

0142905

-29-

In the process of the present invention, a key feature is the development of an extensive, aggressive non-specific cytotoxic attack throughout the tumor cell populaton. Because the cytotoxic attack is deliberately made to be non-specific, care must be taken to insure that this attack is localized within the tumor cell population. In the descriptions above-provided, this localization is achieved through the use of a "cancer-specific" ligand to initiate the process (e.g., to mediate ligand-induced endocytosis in a cancer cell) and prevent such process from occurring on non-cancerous cells. As is well-known, however, the number of singly truly unique ligand-binding receptor sites on cancer cells may be quite limited and some normal, non-cancerous cells throughout the body may similarly express the same particular receptor. In order to prevent the therapy from occurring in areas where such normal cells exist (and where no cancer cells exist), certain steps can, however, be taken.

One means for insuring the localization of the therapy to areas where cancer cells exist is to make the therapy dependent upon more than one "cancer-specific" feature. For example, as described in the preferred embodiment, a cancer-specific ligand is used to induce endocytosis and accumulation of non-metabolizable antigen. Later in the process, a cancer-specific ligand is used to lyse at least some of the cells in which such antigen has been accumulated so as to release the antigen. By choosing these "cancer-specific ligands" to be different, the possibility that a non-cancerous cell will exhibit affinity for both of the ligands is greatly minimized. Thus, even if a non-cancerous cell were to accumulate non-metabolizable antigen by specifically binding its "cancer-specific" ligand carrier, no adverse consequencence would result unless that same cell also exhibited affinity for the later-introduced cancer-specific ligand used to effect lysis of the cell. The same is true for the reverse situation, i.e., lysis of a normal cell is not a problem if that normal cell does not contain accumulated antigen.

Further specificity with respect to localization of the cytotoxic attack only in areas where tumor populations exist can be achieved by requiring even a greater number of cancer-specific interactions to occur before any cytotoxic attack can begin. Thus, in the context of the previously-described embodiments, the process can be arranged such that accumulation of two different non-metabolizable antigens, carried by two different cancer-specific ligands, is required. A third cancer-specific ligand is required to release the accumulated antigens which then complex and precipitate with their specific anti-ligands (previously introduced into the patient in the form of a ubiquitous matrix or as circulating anti-ligands carrying different enzymes). In the embodiment employing enzymatic amplication, the enzymes and substrate are chosen such that both enzymes are required in order to convert the substrate to a cytotoxic material or to a material (e.g., neo-antigen) upon which a cytotoxic attack ultimately is based. A specific example of such a system is the use of lactoperoxidase and glucose oxidase to convert iodide molecules to iodine. Because of the numerous bindings which must occur in order to effect this process, the likelihood that a non-cancerous cell will express all the required receptors is remote.

Another important variation in the previously described embodiments is to eliminate reliance upon ligand-induced endocytosis for accumulation of non-metabolizable antigen. In one method along these lines, a capturing mechanism for non-metabolizable antigen is first introduced into the patient, be it a systemic maxtrix material or circulating antibodies (anti-ligands) against the non-metabolizable antigen (with or without carried enzyme). In either case, the capturing material is designed to capture the non-metabolizable antigen by means of a binding site thereon which is only exposed after the non-metabolizable antigen has de-coupled from a carrier (e.g. monoclonal antibody) therefor. In this embodiment, after the capturing mechanism is in place, a cancer-specific monoclonal antibody, carrying the non-metabolizable antigen, is administered to the patient. The method by which the monoclonal antibody and the non-metabolizable antigen are coupled is such that, when the monoclonal antibody affixes to a specific receptor exhibited by at least some (target) cells of the tumor cell population, the carried non-metabolizable antigen is released. Upon release, the capturing material then captures the non-metabolizable antigen by way of its now exposed antigenic determinant, thereby fixing non-metabolizable antigen either in a matrix material or by means of precipitation occurring through binding to circulating antibody. The radio-labelled antibody which will cause tumor destruction is directed to the nmAG after its intracellular accumulation, release and final fixation to the matrix. It is because the final antibody is not directed against the cancer that one obtains many advantages which are listed in the original document.

Radio-labelled antibodies ( as Fab) against cardiac myosin were infused into dogs after temporary occlusion of a coronary artery which caused cardiac damage. The radio-labelled antibody accumulated in the damaged area of the heart because it attached to the myosin after its release from the damaged cardiac cells. This cardiac imaging is a good model for some critical aspects of the cancer therapy method of the invention:

a) the model shows that an antibody can react with a component after its release from its intracellular location;

b) the accumulation of isotope in the damaged area was 80 to 100 times that of the normal heart;

c) the isotope remained in location for several days;

d) a positive image of the damaged area could be obtained within 6 to 10 minutes after infusing the radio-label.

It is evident that the question arises the damaged heart muscle, acting as a tumor model, could have received a "therapeutic dose" of radiation without systemic damage. In the "cardiac case" the labelled antibody was an Fab fragment with a molecular weight of 56,000. The antibody of the method of the invention can be smaller and reagents other than antibodies can serve the same purpose, but be so small as to virtually eliminate systemic damage.[11] In either case, the now stably affixed non-metabolizable antigen can be used, in all the methods earlier discussed, as the basis for generating an extensive, prolonged cytotoxic attack in the tumor cell population.

One method for bonding monoclonal antibody and non-metabolizable antigen in a way such that the latter is released when the monoclonal antibody binds to a cell receptor is described in published Danish Patent Application No. 1130/83 of March 7, 1983, the text of which is incorporated herein by reference. According to

---

[11]    Khow et al, Journal of Clinical Investigation, Vol. 58, P. 439 (1958). Khow et al, Hybridoma, Vol. 3, No. 1, P. 11 (1984).

this method, the bonding or attachment is accomplished via linkers which are susceptible to cleavage by one or more of the activated complement enzymes.

In another particular embodiment of the present invention, not dependent upon ligand-induced endocytosis, an immuno-specific ligand is coupled to an enzyme (e.g., chondroitinase) which is capable of converting a substrate into a neo-antigen. In this case, the substrate is proteoglycan which is naturally present around cells. The ligand and enzyme are introduced into the patient along with an inhibitor for the enzyme so as to prevent it from acting non-specifically throughout the body. For the enzyme chondroitinase, suitable inhibitors are heparin or keratin sulfate. The ligand and enzyme will affix to at least some (target) cells within the tumor cell population, after which unbound ligand and/or enzyme is eliminated. thereafter, the inhibitor itself is neutralized (e.g., by addition of protamine), permitting the enzyme to act upon proteoglycan in the vicinity of the target cells to convert it to neo-antigen. Since the neo-antigen is not directly on the cell surface and likely is not recognized by the cell as foreign, this neo-antigen will not be endocytosed and thus will remain in the vicinity of the target cells within the tumor cell population. The neo-antigen can then be captured in a suitable matrix or precipitation mechanism, the many molecules of neo-antigen produced and captured in this manner assuring that a sufficient number of molecules of cytotoxic agent will be brought to bear in the area of the tumor cell population to effect widespread and aggressive killing.

In the foregoing embodiments wherein a precipitate or matrix is used as the stable base from which to generate an aggressive and extensive cytotoxic attack within the tumor cell population, it may be necesssry to inhibit the action of macrophages within the patient so as to prevent them from ingesting and destroying the precipitate before the cytotoxic attack can be generated. This inhibition can be effected in a number of ways. For example, the macrophage system can be paralyzed at the time of precipitate formation. In addition, introduction into the patient of agents which inhibit proteolytic enzyme activity will be useful in preventing degradation of the precipitate. Paralysis of the macrophage system is also useful in preventing the system from metabolizing the various molecules utilized in the therapy, e.g., ligands, non-metabolizable antigens, complexes, etc. Other means for achieving this result include arranging such molecules to be small (macrophages typically ingesting only macromolecules) and preliminarily introducing into the patient macromolecular material, similar in structure to the materials to be employed in the therapy, which can be taken up by the macrophages in a quantity such that the capacity of the macrophages for uptake of the subsequently introduced materials will be substantially lessened.

According to another embodiment of the present invention, an immunospecific agent is employed as a carrier for an enzyme which converts a non-lethal substrate to a cytotoxic agent. The immunospecific agent can be any ligand capable of specifically affixing to at least some of the cancer cells within the tumor cell population and having, insofar as possible, no affinity whatsoever for any non-cancerous cells. The ligand can be, therefore, a suitably prepared monoclonal antibody.

In this method, therefore, monoclonal antibody to which enzyme is complexed, is introduced to the patient and becomes affixed on one or more cancer cells in the tumor cell population. Thereafter, any non-affixed monoclonal antibody and/or enzyme is then eliminated from the patient by means of lymphoresis, plasmaphoresis, use of immunosorbents, etc. At this point, therefore, enzyme is localized within the tumor cell population by reason of being coupled to antibody which, in turn, is coupled to one or more cells in the population.

Upon the addition of a suitable substrate to the patient, the enzyme converts the substrate (otherwise non-lethal) to a cytotoxic material. Thus, although the population per se may contain only a few molecules of enzyme (by reason of the fact that (a) the antibody enzyme complex affixes to only a few (target) cells in the population and (b) only a few molecules of enzyme can be coupled to the monoclonal antibody without altering its specificity), each enzyme molecule is capable of converting the substrate to a cytotoxic agent at a rapid rate (e.g., $10^3$ molecules per second). In this way, many molecules of cytotoxic agent can be brought to bear on the entire tumor cell population, achieving a widespread aggressive attack and killing. As can be seen, the cytotoxic attack is not "cancer-specific" in the sense of specifically acting upon only cancer cells. In this way, the heterogeneity of the cancer cell population is not a limiting factor in the therapy. "Specificity" as such (i.e., as between areas of the patient where no tumor cells exist and areas of the patient where a tumor cell population does exist) is achieved by utilizing a small number of cancer cells as target cells to localize the cytotoxic attack only in areas where cancer cells exist.

In the foregoing embodiment, the cytotoxic attack need not be generated directly by the enzyme acting on a substrate. For example, the enzyme can be used to convert a substrate to many molecules of a stable precipitate to which a correspondingly large number of molecules of a specific antibody, carrying cytotoxic agent, can then be directed. Alternatively, the enzyme can be used to convert a substrate to a large number of molecules of a neo-antigen, to which a large number of cytotoxic agent-carrying, specific antibody molecules can be directed. So long as enzyme is localized within the tumor cell population, the enzyme can then be employed to generate, directly or indirectly, an extensive cytotoxic attack throughout the tumor cell population.

In the foregoing embodiments, the enzyme-carrying monoclonal antibody (or other suitable immunospecific ligand) must be one which is capable of remaining on the cell surface for a time sufficient ot permit unbound antibody/enzyme to be eliminated, to permit substrate to be introduced, etc. In general, such antibodies will be rare since the predominant tendency of the cancer cell is to endocytose all materials which affix to its surface and, when internalized, to either accumulate such materials, metabolize them, de-complex them, etc. However, the required antibodies can be found. As discussed in further detail in succeeding sections of this description, isolation techniques developed by me and the subject of a number of co-pending U.S. patent applications are capable of screening an enormous number of cells in a very short time, thereby greatly enhancing the ability to isolate even rare cells making antibodies which will remain stably affixed to cancer cell surfaces.

In accordance with further aspects of the present invention, the ability to bring materials to, and eliminate materials from, the patient (e.g., excess antibodies, enzymes, etc.) can be aided by treatment of the patient to increase the rate of capillary permeability. This can best be done by treating particular regions or segments of the patient with vasoactive materials so as not to encounter the adverse consequences associated with an overall increase in capillary permeability throughout the body. Other methods include localized heat treatment, use of responses carrying vasoactive materials, and the creation of localized allergies.

In accordance with the present invention, it is necessary ot identify (or induce) within the heterogeneous tumor cell population one or more exploitable sub-populations having homogeneous characteristics. For example, within a heterogeneous tumor cell population, one or more cancer cells exhibiting a receptor to which a ligand can specifically be bound will constitute such a homogeneous subpopulation. In addition, the exploitable homogeneous characteristic must be one which is, either alone or in concert with other characteristics, unique to cancer cells, thereby insuring that the therapy will be uniquely directed to tumor cell populations and not to cell populations where no cancer cells exist. By "exploitable" is meant a parameter or characteristic which can be used as a basis for developing an extensive, aggressive cytotoxic attack within the tumor cell population. In the foregoing embodiments, such exploitable characteristics are unique cancer cell receptor sites.

It is also possible to treat a tumor cell population to induce the required exploitable areas of homogeneity or micro-homogeneity. One example of such an approach is the phenomenon of gene amplification, whereby cell division results in the presence in each progeny of a greater number of genes, coded for production of a particular product, than in the parent cell. Because of the rapid division of tumor cells and their inherent instability, it has been suggested that tumor cells are more likely to undergo gene amplification than any other cells. Accordingly, utilization of methods known in the art to favor gene amplification can result in some number of the cancer cells in the tumor population exhibiting a greatly amplified quantity of a particular gene, which amplication can be exploited in order to preferentially deliver to such cells materials required to set in motion the subsequent steps required to achieve the extensive and aggressive killing according to the present invention. For example, known techniques may be employed to induce amplification in some cancer cells with respect to the gene responsible for production of folic acid reductase. Methotrexate, which irreversibly binds to folic acid reductase, and which is labelled with a suitable cytotoxic agent, can be added to the host in a concentration low enough not to bind to and damage or kill normal cells but sufficient to cause preferential binding to cells containing a greatly amplified quantity of folic acid reductase. The cytotoxic agent lyses the cells to which the methotrexate has bound (i.e., the target cancer cells containing a large quantity of folic acid reductase), causing the release from such cells of folic acid reductase. In the presence of added antibody therefor, the released folic acid reductase can be caused to form a precipitate with its antibody and thereby form the basis for the subsequent steps which lead to killing of all the cells of the tumor population according to the method of the present invention. In this embodiment, a "natural" accumulation of material (folic acid reductase) occurs in the target cells, which material, upon the killing of the target cells, is released and causes stable, precipitate formation in the tumor cell population.

It should be apparent from the foregoing that the methods described present essentially an "all-or-nothing" approach in the sense that the steps are designed to localize treatment only to the immediate vicinity of tumor cells and populations by requiring that a number of actions and interactions occur. Thus, for example, in the preferred embodiment of the invention, accumulation of non-metabolizable antigen by any cell alone is not sufficient to effect the therapy. The non-metabolizable antigen must be released from the cell in which it has accumulated (effected via a cancer-specific lysis). By the same token, lysis of any cell alone is insufficient since it is also required that all cells be lysed which accumulated non-metabolizable antigen.

This approach is highly desirable since the cancer therapy of the invention is predicated upon an eventual aggressive, extensive, inherently non-specific cytotoxic attack. Unless a high degree of specificity localizing such attack to tumor cell populations is attained, such an aggressive attack would have obvious untoward effects on normal, non-cancerous cells.

At the same time, however, the high degree of specificity required and the means by which it is achieved necessarily have the capability of greatly limiting the actual number of "spots" or "areas" within the tumor cell population from where the therapeutic, aggressive cytotoxic attack will emanate (e.g., target cells taking up non-metabolizable antigen will be few in number and those which will later be lysed to release non-metabolizable antigen will be even fewer in number).

The aggressiveness, extensiveness and non-specificity of the eventual cytotoxic attack is per se capable of dealing with the fact that it may only emanate from a few areas in the tumor population. However, it is specifically contemplated by the present invention that the number of areas from which the cytotoxic attack proceeds can be increased, without loss of tumor population specificity, by simply conducting a number of the processes set forth herein in parallel. For example, as applied to the preferred embodiment of the invention, a number of different antibodies and the same (or different) non-metabolizable antigen can be employed simultaneously or substantially simultaneously in the first step to effect, in a number of target tumor cells, accumulation of non-metabolizable antigen. Thus antibody no. 1 coupled to non-metabolizable antigen no. 1 will result in accumulation of antigen no. 1 in those few target tumor cells which exhibit a specific receptor for antibody no. 1 capable of mediating endocytosis. At the same time, administration of antibody no. 2 coupled to non-metabolizable antigen no. 1 will result in accumulation of antigen no. 1 in a number of other target tumor cells exhibiting a specific receptor for antibody no. 2. This can be done for any number of different antibodies coupled to, e.g., non-metabolizable antigen no. 1. As a result, the absolute number of target cells containing the non-metabolizable antigen is increased while maintaining tumor population specificity (since each accumulation is mediated by a tumor-specific antibody/receptor interaction. When steps are taken to lyse tumor cells, the absolute number of lysed cells releasing non-metabolizable antigen also is increased, thereby depositing non-metabolizable antigen in an increased number of places (captured by suitable matrix material) throughout the population. Since this capture is the basis for the subsequent cytotoxic attack, a much more aggressive, extensive attack can be brought to bear throughout the tumor population than can be achieved using only one antibody/non-metabolizable antigen. Since the non-metagolizable antigen is not cytotoxic, there is no increased burden on the patient as a result of these variety of complexes circulating throughout the body for some period of time. As earlier discussed, accumulation in the kidney or RES of non-metabolizable antigen can be handled just as in the case of a single antibody/non-metabolizable antigen therapy system.

In the process of the present invention, it will be seen that a variety of ligands are employed as carriers for, e.g., enzymes, cytotoxic agents, materials to be accumulated within cells and the like. As earlier noted, it often will be desirable to employ a number of different ligand materials in a particular therapy in order to increase the specificity of the therapy as to cancer versus non-cancer cell populations. In addition, ligands having particular characteristics are often required in the therapeutic approach of the present invention, for example, ligands which affix to cell surfaces and are not internalized by the cell; ligands which mediate ligand-induced endocytosis; ligands which can be de-coupled from a cell receptor site when internalized by the cell; ligands which do not result in modulation of the cell receptor site; and the like.

To satisfy this desirable need for a repertoire of ligands, it might be possible to prepare such ligands through chemical synthesis. However, it generally will be found that the detailed analysis of the chemistry of cell binding sites and the complex syntheses required to proceed in this manner are prohibitive in terms of time and cost.

A far more desirable means for obtaining such ligands is simply to isolate them from mammalian cells in which they are naturally produced.  To some degree, the art already has occupied itself in this field in the harvesting and isolation of antibodies produced by mammalian cells in response to a diseased state or an administered antigen or organism.  The use of natural antibodies as ligands diminished the possibility that the patient receiving the therapy according to this invention will perceive the liands as foreign and seek to mount an immune attack against them.  In addition, the use of natural antibodies potentially provides an inexhaustable source of the variety of ligands required for therapy.

Ideally, many forms of cancer can be pre-characterized in the sense that a number of potential immunospecific antibodies for delivering agents for endocytosis, for effecting lysis of target cells, etc. can be already available for any particular cancer.  Even more ideally, a patient for whom therapy is proposed can be tested so that the therapy can be individualized for the particular patient, e.g., a number of immunospecific antibodies for the patient's particular cancer cells can be generated.

The difficulty with the foregoing ideals, however, is that the isolation of particular antibody-producing cells making antibody having particular characteristics may involve an enormous number of screenings and isolations which, employing current "state-of-the-art" techniques, will require an enormous amount of time.  Thus, for example, in order to identify and isolate a cell producing antibody which is tailored to a particular cancer and which possesses other specific characteristics, it may be necessary to inoculate mammals with a wide variety of antigens or organisms; to employ a variety of mammals; to employ a variety of inoculation regimens, etc.  If it is desired to perform such testing for a particular patient awaiting therapy, time obviously is of the essence and the luxury of investigating all possible variables simply is not available.

However, in my co-pending U.S. patent applications Serial Nos. 325,051 (filed November 25, 1981), 443,191 (filed November 23, 1982), 460,819 (filed January 25, 1983), each entitled "Method For Isolating Cells", and Serial No. 536,006 (filed September 26, 1983), entitled "Method And Apparatus For Isolation Of Desired Cells By A Thin Layer Of Gel Material", there are described cell isolation techniques which are ideally suited for achieving the rapid isolations which permit the objects of the present invention to be attained in a practicable manner. More importantly, the isolation techniques which are the subject of my co-pending applications, incorporated herein by reference, are predicated upon immunologically specific interactions between desired cellular products (e.g., antibodies) and anti-products (e.g., antigens) thereto. As such, the techniques can be used not only for achieving rapid cell isolations per se, but also for arranging these isolations in a way which aids in the generation of antibodies particularly suited to specific cancer-associated antigens.

The isolation techniques particularly useful in the present invention utilize the interaction of (a) antibody produced by a cell with (b) the antigen to that antibody, to either directly or indirectly bring about a distinguishable environment only in the vicinity where such interaction occurs. The environment can involve the occurrence of a particular condition or the lack of occurrence of a particular condition in the vicinity of the cells making the desired antibody.

The particular means by which these isolations can be brought about are many. For purposes of illustration, some of these methods are described hereinafter as they relate to the isolation of particular, desired antibody-producing cells from a mixed cell population.

Assume for example, the existence of a mixed cell population, such as may result from the harvesting of cells from a mammal to which a particular antigen, antigen-bearing cell or organism has been administered (e.g., cancer cells removed form a patient), containing cells producing a desired antibody product ("desired cells") and cells either not producing any product at all or producing a product which is not desired ("undesired cells"). In one method of isolation, the mixed cell population is arranged in a suitable vessel(s) or other form of apparatus (e.g., glass slide, etc.) such that the cells maintain relatively fixed positions with respect to each other and such that products produced and released from cells have only limited diffusivity or mobility away from the cells from which they were released. After a predetermined period of time, the situation is such that desired cells have in their immediate vicinity a concentration of desired product released therefrom. Other cells in the population either will have no product in their immediate vicinity (because they do not produce and release any products) or will have a concentration in their immediate vicinity of product other than the desired antibody product. The limited mobility of products in the vessel insures that undesired cells will not have desired products in their vicinity. There is then added to the population the antigen specific to the desired antibody and which has coupled thereto a drug lethal to any cell in which it is taken up. Due to the specific interaction of antigen with the high concentration of desired antibody product in the immediate vicinity of desired cells, however, the antigen and drug are prevented from being taken up by desired cells. In all other cells, the antigen is free to pass, with its drug, into the cells and eventually kill them. Thus, after a predetermined period of time, the only remaining living cells will be the desired cells sought to be isolated.

In another method, the cell population is similarly arranged in a suitable vessel along with numerous "scavenger cells" which exhibit the antigen specific to the desired antibody sought. The scavenger cells have the property of being capable of a large, rapid uptake of molecules from their surrounding environment when an antigen/antibody interaction occurs on their surface. Also, the scavenger cells are such that they can be arranged eventually to die after a suitable period of time. Irradiated tumor cells exhibiting particular antigen are suitable scavenger cells. There is then added to the cell population a lethal drug capable of killing cells. However, in the immediate vicinity of desired cells, the drug is rapidly taken up by the scavenger cells by reason of the fact that, in the immediate vicinity of the deisired cells, the antigens on the scavenger cells have interacted with the specific antibody therefor being produced by the desired cells. In the vicinity of all other cells (the undesired cells), no such antibody/antigen interaction occurs and the scavenger cells do not take up the lethal drug at a rate sufficient to prevent death of the undesired cells of the population. After a predetermined time, therefore, the only remaining living cells in the population are desired cells.

In another embodiment, the mixed cell population similarly is arranged in fixed relative position in admixture with cells or carrier bearing the antigen specific to the desired antibody. After a suitable time has been allowed for desired antibody/specific antigen interactions to occur (the interactions being only in the vicinity of desired cells), there is added to the cell population a product which will exhibit specific binding affinity only for desired antibody product or to the complex of desired antibody product and its antigen. This product has affixed to it an enzyme capable of converting a substrate to some different material. As a result of the foregoing (after removal of any unbound enzyme in the system), enzyme remains selectively immobilized in the population only in the immediate vicinity of desired cells. There can then be added to the population any number of substrates which can be converted by the enzyme to any distinguishable condition which will pinpoint the location of desired cells. For example, the enzyme can convert a colored substrate to a colorless material or vice-versa or convert a non-fluorescent substrate to a fluorescent material, etc. Since the conversion is limited to the area where desired cells exist, the distinguishable condition brought about by the conversion can be used as a marker of the location of desired cells. Another "condition" which can be brought about enzymatibally is the addition to the population of a tozic compound which is converted by the enzyme to a non-toxic compound in the vicinity of desired cells or a system in which the enzyme can be used to make a chemical which rescues cells in its vicinity from an environment which otherwise would be lethal.

Enzymatic conversion also can be used to bring about effects only in the vicinity of undesired cells. For example, using the same arrangement as above-described, there is added to the population an enzyme-coupled product exhibiting affinity for the antigen borne on the carrier cells or particles. In the vicinity of desired cells, all antigen on the carrier cells or particles is bound to desired antibody and thus will not bind to the added enzyme-coupled product. The enzyme-coupled product will, however, bind to antigen in the vicinity of all undesired cells since the antigen is there unbound. With the enzyme then immobilized in the vicinity only of undesired cells (all unbound enzyme being removed from the population) enzymatic conversion of a substrate can be used to bring about conditions (or absence of conditions) which mark the location of desired cells.

Preferred isolation techniques make use of porous beads (e.g., of materials such as agarose, alginate, polylysine or combinations thereof) in which individual cells of the cell population are encapsulated along with other materials required for the isolation. In this manner, distinguishable conditions can be brought about in only those beads containing desired cells making desired antibody product.

In one such method, for example, individual cells of a mixed cell population are encapsulated within porous beads along with, e.g., red blood cells having thereon the antigen specific to the desired antibody product sought. The beads are sized so as to retain cells therein while permitting ingress and egress of certain materials. After a predetermined time, therefore, beads containing desired cells contain red blood cells on which the antigen affixed thereon has coupled to the desired antibody product produced by the desired cells. In all other beads (containing undesired cells), no such coupling occurs. If complement is then added to the entire bead population, and the bead population suspended in a suitable medium, the complement will lyse those red blood cells bearing the antigen/antibody complex (i.e., only red blood cells in desired cells). As a consequence, the lysed red blood cells then escape from the beads and cause the beads to be distinguishably lighter and less dense than beads which still contain intact red blood cells (i.e., beads containing undesired cells). The beads containing desired cells will therefore levitate in the bead suspension to a distinguishable level higher than beads containing undesired cells.

Many other variations of these techniques exist wherein some condition can be arranged to occur (e.g. enzymatically) either in beads containing desired cells or beads containing undesired cells so as to bring about a distinguishable state (e.g., weight, color, fluorescence, etc.). Where the condition brought about is the death of undesired cells, beads containing desired cells can be isolated with time since the cells therein will continue to grow and divide and eventually weight the bead to a point where it drops to a distinguishable location in the suspension. Other conditions can be employed, for example, to arrange for the selective dissolution of either beads containing desired cells or beads contining undesired cells. Steps also can be taken to amplify the condition, brought about or initiated by the interaction of specific desired antibody produced by a desired cell with its specific antigen, to facilitate distinguishing desired cell locations or beads containing desired cells.

As applied to the present invention, these isolation techniques offer a means whereby rapid isolation of particular cells in large cell populations can be effected. This is particularly important in the context of the present invention since, in an effort to generate and locate mammalian cells producing particular antibodies to a particular antigenic material, numerous immunizations, hybridizations and isolations are contemplated.

As applied specifically to the therapeutic approach of the present invention, the foregoing isolation techniques permit the rapid screening of a substantial number of cells in an effort to find those making products having particular desired characteristics. It will be noted that many of the illustrated isolation techniques employ antigen "specific" to the desired antibody in the sense that the particular antigen in question has been previously identified and isolated. While this may be the case for some antigens associated with cancer, the general scheme of things will be such that the particular tumor antigens are unknown and incapable of being isolated from the tumor cells. In these cases, the tumor cells per se are employed as the various target cells in the isolation techniques; the isolations identify those cells in the cell population which are making antibody which exhibits affinity for the tumor cell or which lyse the tumor cell, etc. Other suitably chosen target cells and isolation conditions can be chosen so as to isolate cells producing antibodies having other particular characteristics such as the ability to be de-coupled from a receptor or non-metabolizable antigen at specified pH, etc.

A unique case is the desire to find antibodies which do not result in modulation of the receptor site specific therefor on a cancer cell. The isolation of a desired cell producing a particular antibody requires interaction between the antibody and the target cell, followed by some means to amplify the interaction in a way which brings about a distinguishable condition in the vicinity of the desired cell. If the receptor on the target cell disappears after endocytosis of the antibody occurs, it is not possible to determine whether the isolation was negative (i.e., antibody specific to the receptor site was not produced) or positive (i.e., antibody specific to the receptor site was produced, but was internalized without reappearance of the receptor). According to the present invention, this potential difficulty can be solved in the following way.

In a first isolation, the target cells employed are tumor cells (e.g., those of the patient in question) which have been drugged so that no endocytosis can occur, i.e., the receptor sites on the tumor cell are essentially fixed but still capable of binding. Positive isolations from this step will include all cells in the cell population making antibody against any receptor on the tumor cell.

In a next isolation, the population of these positive cells is then re-tested in the presence of normal cells. Positive isolations from this test will include all cells making antibody to receptors on normal cells. Negatives in this test, however, will include those cells making antibody against tumor cell receptors but not against normal cell receptors, and thus are uniquely tailored to cancer cells.

If the population of these negative cells is then tested in the present of the tumor target cells, this time un-drugged, the positives in this test will be those cells making tumor-specific antibody and wherein such antibody does not result in modulation of the receptor site.

Also useful in the practice of the present invention as a means for performing the many rapid isolations which may be required to find cells making antibodies of particular characteristics is the invention described in my co-pending U.S. patent application Serial No. 536,007, filed September 26, 1983, and entitled "Process.For Producing Antibodies". According to that invention, means for producing the full repertoire of antibodies capable of being produced aginat a multi-antigenic material are disclosed. Emphasis is placed therein on means for preventing the continuous re-isolation and re-recovery of antibodies against particularly dominant antigenic sites to the exclusion of antibodies against more obsure sites.

Once cells making a desired antibody of particular characteristics are isolated, it is then necessary to culture the cells to permit continuous production of the antibody. If the isolated cells are not hybird cells, they can be hybridized to a cell line capable of continuous division and then cultured in vivo or in vitro. A particularly preferred culturing system is that described in my U.S. Patent Nos. 3,964,467 and 4,064,006 and in my co-pending U.S. Patent Applications, Serial Nos. 449,779 (filed December 14, 1982) and 450,056 (filed December 15, 1982), wherein a flowing culture medium, preferably comprising lymph, is employed.

In the therapeutic approach to cancer described herein, the toxic agent utilized to effect the widespread aggressive killing of cancer cells preferably is a radioactive material, particularly one which is a gamma-emitter since the gamma particles are capable of penetrating significant distances and therby extend the killing effect to the whole of the tumor cell population. It also is possible to employ materials such as Boron-10, which can be made to release alpha particles upon bombardment with thermal neutrons. Although alpha particles penetrate only short distances, use of the method of this invention to distribute a large number of Boron-10 molecules throughtout the tumor cell population enables the release of such a large number of alpha particles that aggressive killing and extensive killing in the tumor cell population nevertheless can be achieved.

Current immunotherapeutic approaches to cancer are plagued by significant shortcomings which are inherent in the approaches themselves. The desire to continually seek "magic bullets" which will specifically zero in on cancer cells to destroy them while leaving all other cells unaffected is a laudable goal, but is impractical in the context of current knowledge of cancer mechanisms because the heterogeneity and plasticity of cancer cell populations makes it compleely unlikely that any one (or even a few) immunospecific antibodies or ligands will be capable of specifically affecting or binding to every cancer cell in the population. Moreover, current methodolgies for obtaining the array of ligands or antibodies required to deal with all the varieties of cancers and the heterogeneity within each such variety are inadequate or non-existent.

In the approach taken in accordance with the present invention, the therapy is not dependent upon finding a particular characteristic, unique to the cell population in question, on every cell in the cell population. Rather, particular cells in the population are utilized as target cells which serve to localize the therapy to the area of the tumor cell population. Once localized, steps are taken to build from the target cells an aggressive, amplified and widespread therapeutic effect throughout the population, not dependent upon the ability to find particular characteristics on every cell in the population.

It will be recognized that the described therapeutic approach to cancer (applicable, of course, to any other disease) is a significant departure from conventional therapy approaches in a number of key, fundamental respects. One key feature of the invention is that the ultimate cytotoxic step is not "anticancer", i.e., it does not directly depend upon any interaction with a cancer cell. As a consequence of this fact, the cytotoxic agent does not have to consititue an antibody or have its delivery mediated by an antibody. From this fact proceeds the important consequence that the ultimate cytotoxic agent (whether alone or in conjunction with a carrier) can be made small, and therefore be made to travel rapidly through the patient (and, if it does not become affixed, be rapidly excreted by the patient), greatly reducing the burden on the patient by prolonged exposure to circulating poisons. This cannot be done for antibody or antibody-coupled cytotoxic agents since any attempt to make such materials small results in a significant reduction in their specificity and/or binding affinity. As such, they cannot easily be specifically directed to a particular cell and cannot compete with or displace natural antibodies already in the patient having high binding affinity. In the present invention, the final cytotoxic agent need only exhibit specific affinity for some site on a fixed non-metabolizable antigen (or neo-antigen, etc.) rather than specific affinity for a cell receptor. As such, this portion of the process is no more than a chemistry-like problem rather than a complex cancer-specific problem, enabling the use of any number of natural or synthesized molecules for use in the cytotoxic step which can be made small, etc.

It will be apparent to those skilled in the art that the various steps of the process of the present invention can be "tracked" by means of appropriate radioactive- or fluorescein- labelling of reagents used in the process. Indeed, the process of the invention (either in whole or in part) can be used very effectively for diagnostic purposes. For example, use of a radio- or fluorescein- labelled non-metabolizable antigen carried by a cancer-specific antibody (or antibodies) can be used to accumulate labelled antigen in target tumor cells. As a result of the long half-life of accumulated non-metabolizable antigen in the target cell, ample time is available for removing non-accumulated labelled antigen from the patient's bloodstream and for dealing with any accumulation which may have occurred in the kidney or RES. This luxury is not afforded by conventional labelled-antibody imaging systems which must, therefore, view the images while a substantial quantity of label is still present in non-cancerous tissues, in the bloodstream, etc.

CLAIMS

1. A substance for the in vivo treatment of a diseased cell population in a patient comprising an immunological complex which is adapted to serve as a precursor of a cytotoxic attack on a target cell of a cell population of heterogeneous diseased cells of a patient by being internalized by a disease cell by endocytosis

a carrier substance which is immunospecific to a target diseased cell of the diseased cell population, and

a non-metabolizable antigen complexed to the carrier substance, the non-metabolizable antigen being formed of antigenic material which is non-cytotoxic to a target cell, which is not broken down by any system or material to which it is exposed in a patient, and which is adapted to be de-coupled when the complex is internalized within the target cell, whereby the non-metabolizable antigen is adapted to be accumulated in a target cell by endocytosis induced by the carrier substance.

2. A substance in accordance with claim 1 in which the carrier substance is an immunospecific ligand.

3. A substance for the in vivo treatment of a diseased cell population in a patient comprising a material for capturing non-metabolizable antigen material when released from target disease cells in a population of disease cells in a patient comprising a substantially systemic matrix material which is non-cytotoxic and adapted to be infused into a patient for the covalent incorporation into the complex extracellular matrix of the body of the patient, the substantially systemic matrix material exhibiting specificity for the non-metabolizable antigen.

4.    A substance in accordance with claim 3 and further comprising a constituent attached to the material for capturing which is capable of attaching to a specific binding site contained on the non-metabolizable antigen.

5.    A substance for the _in vivo_ treatment of a diseased cell population in a patient comprising a material for establishing a cytotoxic environment adjacent to a matrix within the extracellular material of a patient to which non-metabolizable antigen lysed from a diseased cell of a population of diseased cells is bound comprising a ligand exhibiting specific affinity for an unoccupied determinant site on the non-metabolizable antigen and a cytotoxic agent coupled to the ligand.

6.    A substance for establishing a cytotoxic environment in accordance with claim 5 in which the relative size of the ligand enables it to pass rapidly through the capillaries of a patient to adjacent the non-metabolizable antigen.

7.    A substance for the _in vivo_ treatment of a diseased cell population in a patient comprising an immunological complex including a non-metabolizable antigen and an immunological ligand exhibiting binding affinity therefor, said immunological ligand having coupled thereto a material which is capable, directly or indirectly, of effectuating a cytotoxic effect.

- 57 -

0142905

8. A substance in accordance with claim 7, wherein said material is an enzyme used to convert a substrate to a neo-antigen such that antibody specific to the neo-antigen can then be employed to carry molecules of a cytotoxic agent to a diseased cell population.

9. A substance for the in vivo treatment of a diseased cell population in a patient comprising a material for forming a precipitate within the extracellular material of a patient to which non-metabolizable antigen is released from a lysed diseased cell of a population of diseased cells comprising a ligand exhibiting specific affinity for an unoccupied determinant site on the non-metabolizable antigen and an enzyme coupled to the ligand, the enzyme being adapted to be bound to the non-metabolizable antigen in response to the ligand to form a precipitate.

10. A substance for the in vivo treatment of a diseased cell population in a patient comprising a ligand material which exhibits substantially specific binding affinity for a receptor, unique to the diseased cells, exhibited by at least some target cells in the diseased cell population, the ligand material having coupled thereto an enzyme which is capable of converting a substrate into a neo-antigen when the ligand material and enzyme are introduced into a patient along with an inhibitor for the enzyme to prevent the enzyme from acting throughout the body of the patient.

Fig.1.

Fig.2.

Fig.3.

Fig.4.

Fig.5.

Fig.6.

Fig.7.